# EUROPÄISCHE PATENTSCHRIFT

(11) **EP 2 276 422 B1**
(45) Veröffentlichungstag und Bekanntmachung des Hinweises auf die Patenterteilung: **21.02.2018**
(21) Anmeldenummer: 09729931.7
(22) Anmeldetag: 07.04.2009
(51) Int. Cl.: A61F 2/46, A61F 2/36, A61F 2/30

(54) **MODULARE FEMURKOPFPROTHESE**
MODULAR FEMORAL HEAD PROSTHESIS
PROTHÈSE DE TÊTE FÉMORALE MODULAIRE

(30) Priorität: 07.04.2008 DE 202008004730 U
(43) Veröffentlichungstag der Anmeldung: 26.01.2011
(73) Patentinhaber: Waldemar Link GmbH & Co. KG, 22339 Hamburg (DE)
(72) Erfinder: KELLER, Arnold, 23863 Kayhude (DE)
(74) Vertreter: Glawe, Delfs, Moll
(86) Internationale Anmeldenummer: PCT/EP2009/002561
(87) Internationale Veröffentlichungsnummer: WO 2009/124719

(56) Entgegenhaltungen:
- EP-A1- 0 283 706
- WO-A1-96/01086
- DE-A1- 3 535 158
- FR-A1- 2 735 971
- FR-A1- 2 735 972
- US-A1- 2007 123 908
- US-B1- 6 330 845

## Beschreibung

Die Erfindung betrifft eine modulare Femurkopfprothese umfassend einen Schaftteil zum Einsetzen in eine meduläre Öffnung des Femurs und einen Halsteil mit einer Aufnahmefläche, der auf eine Stützfläche am proximalen Ende des Schaftteils mittels einer Verschraubung befestigbar ist. Bei aufgrund von Verschleiß oder Erkrankungen auftretenden Defekten im Bereich des Kopfes des natürlichen Femurs und bei Implantation eines künstlichen Hüftgelenks ist eine Femurkopfprothese indiziert. Verschiedene Ausführungsformen für Femurkopfprothesen sind bekannt. Sie unterscheiden sich insbesondere hinsichtlich der Art ihrer Verankerung im Femur. Genauer gesagt betrifft der Unterschied die Form und insbesondere die Länge der Schäfte. Die Auswahl des jeweiligen Schafts und seiner Länge erfolgt in Anpassung an die individuellen anatomischen Verhältnisse des Patienten. Um die Zahl der erforderlichen verschiedenen Varianten gering zu halten, ist es bekannt, die Femurkopfprothese modular aufzubauen. So ist eine Ausführung bekannt, bei der ein Schaftteil zur Verankerung in einem medulären Kanal des Femurs und ein Kopfteil, welches die Gelenkkugel zur Bildung eines Hüftgelenks trägt, mittels einer Dehnschraube verbunden sind (EP-A-0 845 251). Mit dieser modularen Femurkopfprothese kann bereits eine beträchtliche Verringerung der erforderlichen Anzahl der Teile erreicht werden. Jedoch sind immer noch verhältnismäßig viele Teile erforderlich, um individuelle Unterschiede in Bezug auf die erforderliche Schaftlänge und/oder Höhe des Kopfteils berücksichtigen zu können. EP-A-0 283 706 beschreibt eine Hüftprothese, wobei die sich am Knochen abstützende Querfläche der Prothese von einer Mehrzahl von mit Zwischenraum voneinander in den Knochen eingreifenden Stützteilen gebildet ist. Diese Stützteile sind zweckmäßigerweise so ausgebildet, daß sie sternartig von einem Ring, einer Hülse oder dergleichen ausgehen. Der zwischen den Stützteilen gebildete Zwischenraum ermöglicht es, daß eine Knochenverbindung zwischen den unterhalb und oberhalb der Stützteile befindlichen Abschnitte des Oberschenkelknochens verbleibt oder wieder entsteht.

Der Erfindung liegt die Aufgabe zu Grunde, eine modulare Femurkopfprothese der eingangs genannten Art dahingehend zu verbessern, dass sie mit weniger Teilen auskommt. Die erfindungsgemäße Lösung liegt in einer Femurkopfprothese mit den Merkmalen der unabhängigen Ansprüche. Vorteilhafte Weiterbildungen sind Gegenstand der abhängigen Ansprüche.

Bei einer modularen Femurkopfprothese umfassend einen Schaftteil zum Einsetzen in eine meduläre Öffnung des Femurs und einen Halsteil mit einer Aufnahmefläche, der auf einer Stützfläche am proximalen Ende des Schaftteils mittels eines Verschraubungselements befestigbar ist, ist gemäß der Erfindung mindestens ein gesonderter Zwischenring vorgesehen, der zwischen der Stützfläche des Schaftteils und der Aufnahmefläche des Halsteils angeordnet und mittels des Verschraubungselements fixiert ist. Ferner ist die Aufnahmefläche versenkt in einer den Zwischenring kammernden Vertiefung angeordnet und das Verschraubungselement ist eine Dehnschraube. Die Erfindung erreicht mit der Anordnung des Zwischenrings zwischen dem Schaftteil und dem Halsteil eine bessere Anpassbarkeit der Femurkopfprothese an die individuellen anatomischen Erfordernisse des Patienten, wobei dank der Kammerung des Zwischenrings in der Vertiefung eine hohe Verbindungssicherheit erreicht wird. Mit der Verwendung des erfindungsgemäßen Zwischenrings kann die wirksame Schaftlänge der Femurkopfprothese verändert werden, ohne dass eine Mehrzahl an Schaftteilen mit unterschiedlichen Längen erforderlich ist. Dem Chirurgen ist damit eine Möglichkeit an die Hand gegeben, auch intraoperativ die wirksame Schaftlänge der Femurkopfprothese zu variieren, ohne dass dazu ein anderes Schaftteil erforderlich ist. Damit ist ihm Flexibilität an die Hand gegeben, auch während der Operation effizient auf veränderte Anforderungen reagieren zu können, beispielsweise wenn Knochendefekte umfangreicher sind als bei der Operationsplanung erwartet und damit eine größere Schaftlänge erforderlich ist. Weiterhin bietet die verkammerte Anordnung den Vorteil, dass durch das Ineinandergreifen des Zwischenrings des Schaftteils in die Vertiefung an dem Halsteil eine Führung gebildet ist, welche Schaft- und Halsteil in einer definierten Lage zueinander hält. Die Erfindung vereinigt damit Vorteile hinsichtlich geringerer Teilezahl mit größerer Variabilität, und zwar dank der versenkten Anordnung ohne Einschränkungen hinsichtlich der Stabilität der Verbindung zwischen den Teilen.

Zweckmäßigerweise sind die Stützfläche und die Aufnahmefläche so ausgebildet, dass sie im Wesentlichen eben sind. Unter im Wesentlichen eben wird hierbei verstanden, dass sie frei sind von aufgesetzten Strukturelementen, wie Zentrierungszapfen oder Ähnlichem. Die ebene Gestaltung bietet einerseits den Vorzug, dass sie verhältnismäßig leicht herzustellen ist. Sie bietet aber andererseits den Vorzug, dass sie eine große unterbrechungsfreie Fläche für die Kraftübertragung zur Verfügung stellt. Die Montage ist ebenfalls erleichtert, da es anders als bei aufgesetzten Strukturelementen nicht zu Verkantungen oder Verklemmungen beim Zusammenfügen des Schaftteils und des Halsteils kommen kann. Das Verschraubungselement ist eine Dehnschraube. Unter einer Dehnschraube wird eine Schraube verstanden, deren Schaft in einem Bereich zwischen Gewinde und Kopf eine bezogen auf den Gewindekerndurchmesser reduzierte Weite aufweist. Die Ausführung als Dehnschraube bietet den Vorteil, dass auch bei einem Setzen der Teile des Implantats oder unter dem Einfluss von Dauer- und Schwingbelastungen keine Gefahr einer Lockerung der Verschraubung besteht. Dies ist insbesondere bei Verwendung von mehr als einem Zwischenring von Bedeutung. Die Befestigungssicherheit und die Funktionssicherheit der implantierten Prothese werden durch die Dehnschraube deutlich erhöht.
Vorteilhaft ist die kammernde Vertiefung als eine Hülse ausgebildet, deren Innenmantel das proximale Ende des Schaftteils umschließt und deren Grund die Aufnahmefläche bildet. Damit kann die gesamte Querschnittsfläche des proximalen Endes des Schaftteils als Stützfläche dienen. Es steht eine große lastübertragende Fläche zur Verfügung. Zweckmäßigerweise ist der Außenmantel des proximalen Endes des Schaftteils als Führungsfläche ausgebildet. Unter Führungsfläche wird hierbei verstanden, dass sie so zusammenwirkend mit dem Innenmantel der Hülse ausgebildet ist, dass eine praktisch spielfreie Abstützung gegenüber Kipp- und Biegemomenten gegeben ist.
Vorzugsweise ist weiter vorgesehen, dass das Verschraubungselement einen Gewindebereich aufweist, dessen Länge größer ist als die Stärke des Zwischenrings. Damit wird erreicht, dass ein und dasselbe Verschraubungselement verwendet werden kann, unabhängig davon, ob in dem konkreten Anwendungsfall ein Zwischenring verwendet wird oder nicht. Es braucht dann kein gesondertes Verschraubungselement für die Anwendung der erfindungsgemäßen Prothese mit oder ohne Zwischenring vorgesehen zu sein. Dies ist insbesondere dann zweckmäßig, wenn mehrere Zwischenringe mit unterschiedlichen Stärken vorgesehen sind. Dann kann eine Verringerung der Anzahl der Verschraubungselemente erreicht werden, wenn die Länge des Gewindes mindestens so groß ist wie die Stärke des dünnsten der Zwischenringe.

Zweckmäßig ist es, zwei verschiedene Zwischenringe vorzusehen, deren Stärken im Verhältnis 1:3 stehen. Es können dann durch Auswahl von zwei Zwischenringen fünf verschiedene Längen bereitgestellt werden (ganz ohne, mit einem Zwischenring einfacher Stärke, mit zwei Zwischenringen zweifacher Stärke, mit einem Zwischenring dreifacher Stärke und mit einem Zwischenring dreifacher und einem einfacher Stärke). Eine Verdoppelung der Variationsmöglichkeiten bezüglich der Länge kann erreicht werden, wenn statt zwei Zwischenringen drei Zwischenringe vorgesehen werden, deren Stärke zueinander im Verhältnis 5:2:1 steht. Mit diesen drei Zwischenringen lassen sich sämtliche Stärken zwischen dem Ein- und dem Achtfachen der Stärke des dünnsten Zwischenrings einstellen. Zusammen mit der Variante, dass überhaupt kein Zwischenring vorgesehen ist, ergeben sich bei drei Zwischenringen insgesamt neun verschiedene Längenvarianten. Es versteht sich, dass zweckmäßigerweise nicht nur ein Verschraubungselement als Längenvariation verwendet wird, sondern mehrere Verschraubungselemente in verschiedenen Längen vorgesehen sind. Die Zahl der verschiedenen Teile kann damit weiter verringert werden. Vorzugsweise sind die Verschraubungselemente dabei so ausgelegt, und insbesondere die Gewindelänge so groß gewählt, dass die Anzahl der verschiedenen Verschraubungselemente kleiner ist als die Anzahl der Kombinationen der Zwischenringe.

Vorzugsweise sind an den Stütz- und Aufnahmeflächen ineinandergreifende Verzahnungen vorgesehen. Dank einer solchen Verzahnung können Schaft- und Halsteil in besonderem Maße gegenüber einer unerwünschten gegenseitigen Verdrehung geschützt sein. Zweckmäßigerweise ist dazu die Verzahnung als eine Radialverzahnung ausgeführt. Sie verhindert einerseits zuverlässig eine unerwünschte gegenseitige Verdrehung der beiden Teile, und zum anderen erreicht sie eine gegenseitige Zentrierung.

Vorzugsweise ist ein Momentenstützgriff vorgesehen, der auf lösbare Weise drehfest mit dem Halsteil verbindbar ist. Über ihn kann das beim Anziehen der Verschraubung auftretende Gegendrehmoment an dem Halsteil aufgenommen werden. Eine unerwünschte Einleitung des Gegendrehmoments in den Schaftteil kann damit vermieden werden. Zweckmäßigerweise ist der Momentenstützgriff mit einer Aufnahme für einen Haltekonus des Halsteils versehen. Der üblicherweise zum Tragen einer Gelenkkugel ohnehin vorhandene Haltekonus ist ausreichend kräftig dimensioniert und in der Regel gut zugänglich. Mit der genannten Aufnahme kann der Momentenstützgriff auf einfache Weise an das Halsteil angekoppelt werden. Besonders zweckmäßig ist es, wenn an der Aufnahme eine Klemmeinrichtung mit einem handbetätigten Exzenter angeordnet ist. Dem Operateur ist es so ermöglicht, ohne weiteres Werkzeug den Momentenstützgriff mit dem Halsteil zu verbinden beziehungsweise es auch wieder nach Festziehen der Verschraubung von ihm zu lösen.

Vorzugsweise ist ein drehmomentkalibrierter Anzugschlüssel vorgesehen. Unter drehmomentkalibriert wird hierbei verstanden, dass der Anzugschlüssel auf ein vorbestimmtes Moment einstellbar ist. Besonders zweckmäßig geschieht dies durch einen Zeiger und eine Markierung, wobei das vorbestimmte Moment erreicht ist, wenn der Zeiger auf die Markierung zeigt.

Die Erfindung erstreckt sich weiter auf einen modularen Femurkopfprothesensatz mit mehreren Zwischenringen in verschiedenen Stärken.

Die Erfindung wird nachfolgend unter Bezugnahme auf die Zeichnung erläutert, in der ein vorteilhaftes Ausführungsbeispiel dargestellt ist. Es zeigen:
- Fig. 1: eine Explosionsdarstellung einer modularen Femurkopfprothese gemäß der Erfindung;
- Fig. 2 a-c: drei Beispiele für unterschiedliche Längen der erfindungsgemäßen modularen Femurkopfprothese gemäß Fig. 1;
- Fig. 3: Ausführungsvarianten für Schrauben der modularen Femurkopfprothese gemäß Fig. 1;
- Fig. 4: Zwischenringe der modularen Femurkopfprothese gemäß Fig. 1;
- Fig. 5: eine vergrößerte Darstellung eines Schaftteils mit einem Zwischenring der modularen Femurkopfprothese;
- Fig. 6: eine Aufsicht auf einen Momentenstützgriff zur Verwendung an einer modularen Femurkopfprothese gemäß Fig. 1;
- Fig. 7: eine vergrößerte Darstellung einer Klemmeinrichtung des in Fig. 6 dargestellten Momentenstützgriffs; und
- Fig. 8: ein Anzugschlüssel für die in Fig. 3 dargestellten Schrauben.

Eine modulare Femurkopfprothese gemäß dem in den Figuren dargestellten Ausführungsbeispiel umfasst ein Schaftteil 1 und ein Halsteil 2, die über eine am Halsteil 2 angesetzte und in den Schaftteil 1 eingreifende Dehnschraube 3 als Verschraubungselement miteinander verbunden sind. Die modulare Femurkopfprothese kann ferner einen oder mehrere Zwischenringe 4 aufweisen.

Der Schaftteil 1 umfasst einen unteren, abschnittsweise konisch zulaufenden Verankerungsbereich 11 mit mehreren in Längsrichtung des Schaftteils 1 verlaufenden Verankerungsrippen 12. Weiter umfasst der Schaftteil 1 in seinem oberen Bereich ein einen reduzierten Querschnitt aufweisenden Kupplungsteil 13. Er weist einen im Wesentlichen zylindrischen Mantel 14 auf. Seine Außenkontur stimmt mit derjenigen der Zwischenringe 4 überein. An seinem oberen, proximalen Ende weist er eine Stützfläche 15 auf, die mit einer Radialverzahnung 16 versehen ist.

Der Halsteil 2 ist in zwei Funktionsbereiche untergliedert. Ein erster Funktionsbereich ist ein Haltebereich 21 mit einem als Konusstumpf ausgeformten Haltezapfen für eine Gelenkkugel (nicht dargestellt) eines künstlichen Hüftgelenks. Weiter umfasst der Halsteil 2 einen Verankerungsbereich 22, der sich distal an dem Halsbereich 21 anschließt und sich in Richtung der Längsachse der Femurkopfprothese erstreckt. Der Verankerungsbereich 22 weist an seinem distalen Ende eine Verankerungshülse 24 auf. Ihre Weite und Form sind so bemessen, dass sie mit der Weite und Form des Zwischenrings 4 und des zylindrischen Kupplungsteils 13 übereinstimmen. Die Hülse 24 bildet mit ihrem Grund 25, der etwa rechtwinklig zur Längsachse der Femurkopfprothese verläuft, eine Aufnahmefläche. Sie ist dazu ausgebildet, mit der Stützfläche 15 des Schaftteils 1 zusammenzuwirken. Dazu weist die Aufnahmefläche 25 eine Radialverzahnung auf, die komplementär zu derjenigen des Schaftteils 1 ist. Die Tiefe der Hülse 24 ist so gewählt, dass mindestens der Zwischenring 4 und die Stützfläche 15 im montierten Zustand eintauchen, so dass sich eine Umkammerung ergibt. Durch die Radialverzahnung 16 sowie durch die Umkammerung mittels der Hülse 24 ist eine sichere und positionsgenaue Anordnung der beiden Teile zueinander ermöglicht.

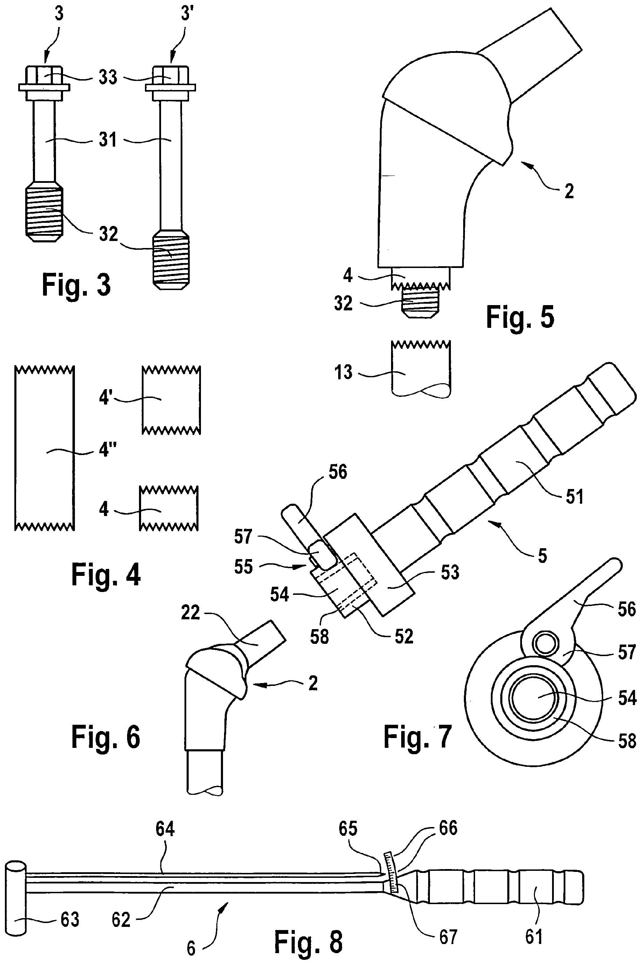

Der Schaftteil 1 weist an seinem proximalen Ende im Zentrum der Stützfläche 15 eine Verschraubungsbohrung 16 auf. Sie ist als ein Sackloch ausgeführt und mit einem Innengewinde versehen, dessen Abmessung auf das Gewinde einer Dehnschraube 31 abstimmbar ist. Damit kann durch einfaches Anziehen der Schraube 31 eine feste und verdrehungsgeschützte Verbindung zwischen dem Schaftteil 1 und dem Halsteil 2 der erfindungsgemäßen modularen Femurkopfprothese erreicht werden.
Weiter ist nicht nur ein Zwischenring 4, sondern vielmehr sind ein Satz von unterschiedlichen Zwischenringen 4, 4' und 4" vorgesehen. Sie unterscheiden sich im Wesentlichen durch ihre unterschiedliche Stärke; die übrigen Außenabmessungen sowie das Vorhandensein von Radialverzahnungen an Unter- und Oberflächen sind vorzugsweise gleich. Die Verschraubungselemente sind als handelsübliche Dehnschrauben 3 mit einem reduzierten Schaftdurchmesser 31 ausgeführt. Am unteren, distalen Ende der Dehnschrauben 3 ist ein Feingewinde 32 ausgebildet. Dessen Länge ist so gewählt, dass mittels nur einer Schraube zwei verschiedene Größen (Längen) der modularen Femurkopfprothese realisiert werden können. Dazu ist vorgesehen, dass die Länge des Gewindes 32 der Dehnschrauben 3 mindestens so lang ist wie der Stärkenunterschied von zwei verschiedenen Zwischenringen 4, 4'. Damit wird erreicht, dass ein und dieselbe Dehnschraube 3 für mindestens zwei verschiedene Größen von Zwischenringen 4, 4', 4" Verwendung finden kann. Damit wird erreicht, dass trotz einer dank der Zwischenringe großen Anzahl von Kombinationen nur eine beträchtlich geringere Anzahl von verschieden langen Dehnschrauben 3, 3', wie in Fig. 3 dargestellt, benötigt wird. Es versteht sich, dass hierbei unter der Länge des Gewindes 32 die tatsächliche Nutzlänge zu verstehen ist, also abzüglich eventueller nicht voll lasttragender Gewindegänge am Anfang beziehungsweise Ende des Gewindes. Die Dehnschraube 3 ermöglicht es, dass die Verschraubung mit einer bestimmten Kraft und damit mit einem bestimmten Drehmoment vorverspannt werden kann. Damit wird der Vorteil realisiert, dass eine gewisse Elastizität in die Verschraubung integriert ist, so dass es auch unter kleinen Bewegungen (sogenannten Micromovements) zwischen Halsteil 2, Zwischenringen 4, 4', 4" und Schaftteil 1 nicht zu Lockerungen der Verschraubung kommt. Um die Dehnschrauben 3 mit dem richtigen Drehmoment anzuziehen, wird vorzugsweise der in Fig. 8 dargestellte Anzugschlüssel verwendet. Er wird später noch näher erläutert werden.

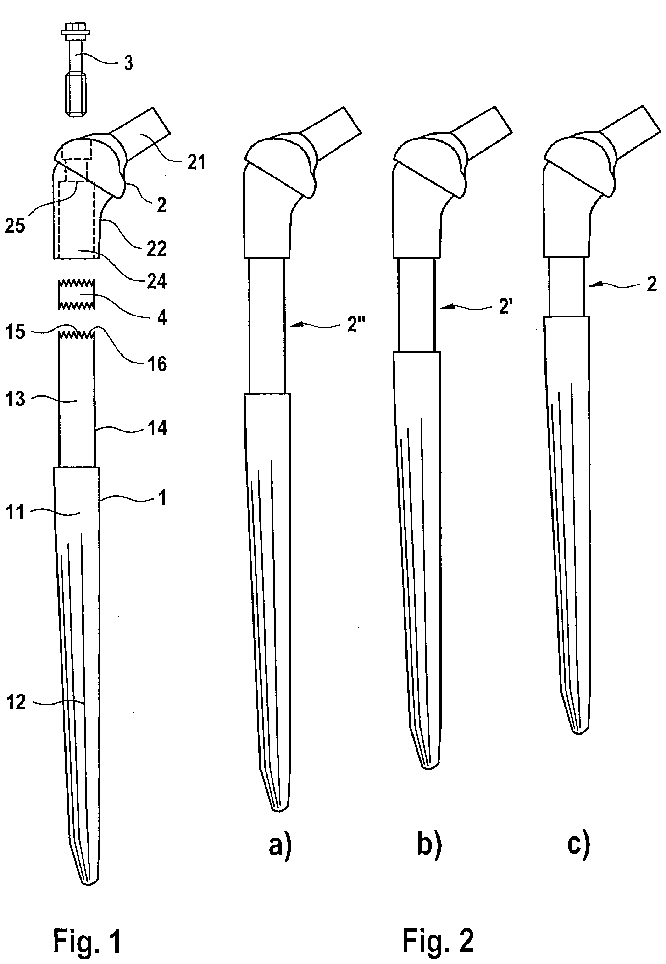

Die in Fig. 4 dargestellten verschiedenen Zwischenringe 4, 4', 4" weisen eine Stärke auf, die einem Verhältnis von 5:2:1 entspricht. Beispielsweise ist der kleinste Zwischenring 4 so bemessen, dass er eine Stärke von 5 mm aufweist, der mittlere Dickenring 4' weist eine Stärke von 10 mm und der größte Dickenring 4" weist dann eine Stärke von 25 mm auf. Mit einer solchen Aufteilung ist es ermöglicht, mit lediglich drei verschiedenen Zwischenringen 4, 4', 4" alle Stärkenmaße von dem Einfachen bis zum Achtfachen der Stärke des kleinsten Zwischenrings 4 zu erreichen, wobei höchstens drei Zwischenringe Verwendung zu finden brauchen und in allen Fällen bis zu einer Stärke von dem Siebenfachen lediglich zwei Ringe erforderlich sind. Damit wird ein nahezu optimaler Kompromiss aus Flexibilität einerseits und Verringerung der Zahl der Zwischenringe 4, 4', 4" andererseits erreicht. Beispiele für so gebildete Femurkopfprothesen 2, 2', 2" unterschiedlicher Länge sind in Fig. 2a, b, c dargestellt.

Während der Operation braucht der Operateur lediglich anhand der konkreten Situation eine gewünschte Länge der modularen Femurkopfprothese zu bestimmen, und entsprechend dieser Länge eine erforderliche Anzahl und Art von Zwischenringen 4, 4', 4" auszuwählen. Die Zwischenringe werden in die Hülse 24 des Halsteils 2 eingeschoben. Dies ist in Fig. 5 dargestellt. Zur Montage wird durch den Zwischenring 4 das Gewinde 34 der Dehnschraube 3 geschoben, bis es aus dem Zwischenring 4 herausragt. Die Dehnschraube wird dann weiter eingedreht, bis das Gewinde 34 frei kommt und der Zwischenring 4 den Schaft 31 umgibt. Das Kupplungsstück 13 kann dann angesetzt werden, und die Dehnschraube 3 mit ihrem Gewinde 34 in die Aufnahmebohrung 16 eingeschraubt werden. Durch Festziehen mittels des Anzugswerkzeugs 6 sind dann Schaftteil 1 und Halsteil 2 fest miteinander verbunden. Soll eine modulare Femurkopfprothese eine etwas größere Länge aufweisen, so braucht lediglich anstelle des Zwischenrings 4 ein Zwischenring 4' mit größerer Stärke vorgesehen zu werden. Da zweckmäßigerweise die Länge des Gewindes 34 größer als die Stärkendifferenz zwischen dem Zwischenring 4 und der nächstgrößeren Dicke des Zwischenrings 4' ist, ragt das Gewinde 34 auch bei aufgeschobenem dickeren Zwischenring 4' noch unten aus dem Zwischenring 4' heraus, so dass es unmittelbar ohne weitere Änderungen in die Aufnahmebohrung 16 des Schaftteils 1 eingeführt und festgezogen werden kann. Lediglich bei größeren Längenvariationen, die ein Verwenden mehrerer oder deutlich abweichender Zwischenringe 4" erforderlich machen, kann es erforderlich sein, dass eine andere Dehnschraube 3' mit größerer Länge (s. Fig. 3) zu verwenden ist.

Zur erleichterten Implantation der zusammengesetzten Femurkopfprothese ist ein Momentenstützgriff 5 vorgesehen. Dieser ist dazu ausgebildet, auf den zur Aufnahme des Gelenkkopfes (nicht dargestellt) vorgesehenen Konus 22 aufgeschoben zu werden, um die beim endgültigen Festziehen der Verschraubung 3 auftretenden Gegendrehmomente besser abstützen zu können. Damit wird erreicht, dass die durch das Verschrauben auftretenden Momente nicht den Schaftteil 1 belasten. Der Momentenstützgriff 5 umfasst einen zum Greifen mit einer Hand ausgebildeten Stiel 51, an dessen distalem Ende 52 ein Aufnahmekopf ausgebildet ist. An dem Übergang zwischen dem Stiel 51 und dem Aufnahmekopf 52 ist ein Bund 53 als Anschlag angeordnet. Im Kopf 52 ist eine Aufnahmebohrung 54 für den Konus 22 des Halsteils 2 angeordnet. Ferner ist an dem Kopf 52 eine Klemmeinrichtung 55 vorgesehen. Sie umfasst einen Klemmflügel 56, der schwenkbar an der distalen Stirnseite des Bunds 53 angeordnet ist, und einen in den Bereich der Aufnahmebohrung 54 ragenden Exzenter 57 aufweist. In der Aufnahmebohrung 54 ist ein Kunststoffbecher 58 angeordnet, der die Seitenwände sowie den Grund der Aufnahmebohrung 54 verkleidet. Der Kunststoffbecher 58 ist flexibel. An seinem äußeren Mantel liegt der Exzenterbereich 57 der Klemmeinrichtung 55 an. Der Exzenterbereich 57 ist so ausgebildet, dass er bei Anklappen des Flügels 56 an den Bund 53 den Kunststoffbecher 58 zusammendrückt. Damit ist der in die Aufnahmebohrung 54 eingesetzte Konus 22 sicher an dem Momentenstützgriff 5 gehaltert. Durch einfaches Zurückbewegen des Flügels 56 bewegt sich der Exzenter 57 in eine Position, in der der Kunststoffbecher 58 nicht mehr gegen den Konus 22 gepresst wird, so dass letzterer freigegeben wird und der Momentenstützgriff 5 leicht abgenommen werden kann.

Um bei der Implantation die Verschraubung 3 auch unter erschwerten Bedingungen sicher mit dem gewünschten Drehmoment anziehen zu können, ist der Anzugschlüssel 6 vorgesehen. Er umfasst einen Handgriff 61, der über einen langgestreckten Torsionsstab 62 mit einer Steckhülse 63 verbunden ist. Sie ist dazu ausgebildet, auf einen Schraubenkopf 33 der Verschraubung aufgesetzt zu werden, um die Dehnschraube 3 zu drehen. Dazu weist die Steckhülse 63 in ihrem unteren Bereich eine hexagonale Öffnung mit einer Weite entsprechend derjenigen des Schraubenkopfes 33 auf. In dem oberen Bereich der Steckhülse 63 ist ein aus Stahl gebildeter starrer Messstab 64 verdrehfest angeordnet. Er läuft parallel zu dem Torsionsstab 62 und trägt an seinem handgriffseitigen Ende eine Zeigespitze 65. Sie wirkt mit einer fest auf dem Handgriff 61 angeordneten Momentenskala 67 zusammen. Letztere weist Markierungen 66 für ein Nullmoment sowie ein gewünschtes Anzugsmoment auf. Der Anzugschlüssel 6 wird in an sich bekannter Weise so verwendet, dass beim Anziehen der Dehnschraube 3 das aufgebrachte Drehmoment durch eine relative Torsion zwischen dem Torsionsstab 62 und dem starren Anzeigestab 64 auf der Skala 67 visualisiert wird. Damit braucht der Operateur nur noch die entsprechende Markierung 66 auf der Skala 67 im Auge zu behalten, um auch bei schwierigen Implantations- beziehungsweise Zugangsverhältnissen eine Gewähr für ein Anzugsmoment in der richtigen Höhe zu erhalten.

## Patentansprüche

1. Modulare Femurkopfprothese umfassend einen Schaftteil (1) zum Einsetzen in eine meduläre Öffnung eines Femurs und einen Halsteil (2) mit einer Aufnahmefläche (25), der auf einer Stützfläche (15) im Endbereich des Schaftteils (1) mittels eines Verschraubungselements (3) befestigbar ist,
wobei
mindestens ein gesonderter Zwischenring (4) vorgesehen ist, der zwischen der Stützfläche (15) des Schaftteils (1) und der Aufnahmefläche (25) des Halsteils (2) angeordnet und mittels des Verschraubungselements fixiert ist, und wobei die Aufnahmefläche (25) versenkt in einer den Zwischenring (4) kammernden Vertiefung angeordnet ist, **dadurch gekennzeichnet, dass** das Verschraubungselement eine Dehnschraube (3) ist.

2. Modulare Femurkopfprothese nach Anspruch 1,
**dadurch gekennzeichnet, dass**
die Stütz- und Aufnahmeflächen (15, 25) im Wesentlichen eben sind.

3. Modulare Femurkopfprothese nach einem der vorangehenden Ansprüche,
**dadurch gekennzeichnet, dass**
die Aufnahmefläche (25) am Grund einer Hülse (24) ausgebildet ist, deren Innenmantel das proximale Ende des Schaftteils (1) umschließt.

4. Modulare Femurkopfprothese nach Anspruch 3,
**dadurch gekennzeichnet, dass**
der Außenmantel (14) des proximalen Endes des Schaftteils (1) als Führungsfläche ausgebildet ist.

5. Modulare Femurkopfprothese nach einem der vorangehenden Ansprüche,
**dadurch gekennzeichnet, dass**
das Verschraubungselement einen Gewindebereich (32) aufweist, der länger ist als die Stärke des Zwischenrings (4).

6. Modulare Femurkopfprothese nach einem der vorangehenden Ansprüche,
**dadurch gekennzeichnet, dass**
mehrere Zwischenringe (4, 4', 4") mit unterschiedlichen Stärken vorgesehen sind.

7. Modulare Femurkopfprothese nach Anspruch 6,
**dadurch gekennzeichnet, dass**
zwei Zwischenringe mit einer Teilung der Stärken im Verhältnis 1:3 vorgesehen sind.

8. Modulare Femurkopfprothese nach Anspruch 6,
**dadurch gekennzeichnet, dass**
drei Zwischenringe (4, 4', 4") mit einer Teilung der Stärken im Verhältnis von 5:2:1 vorgesehen sind.

9. Modulare Femurkopfprothese nach einem der Ansprüche 6 bis 8,
**dadurch gekennzeichnet, dass**
mehrere Verschraubungselemente in verschiedenen Längen vorgesehen sind.

10. Modulare Femurkopfprothese nach Anspruch 9,
**dadurch gekennzeichnet, dass**
die Anzahl der verschiedenen Verschraubungselemente kleiner ist als die Anzahl der Kombinationen der Zwischenringe (4, 4', 4").

11. Modulare Femurkopfprothese nach einem der vorangehenden Ansprüche,
**dadurch gekennzeichnet, dass**
an den Stütz- und Auflageflächen (15, 25) ineinandergreifende Verzahnungen (16) vorgesehen sind.

12. Modulare Femurkopfprothese nach Anspruch 11,
**dadurch gekennzeichnet, dass**
die Verzahnung eine Radialverzahnung (16) ist.

13. Modularer Femurkopfprothesensatz zur Bildung einer modularen Femurkopfprothese nach einem der Ansprüche 1 bis 12,
**dadurch gekennzeichnet, dass**
mehrere Zwischenringe (4, 4', 4") in verschiedenen Stärken vorgesehen sind.

14. Modularer Femurkopfprothesensatz nach Anspruch 13,
**dadurch gekennzeichnet, dass**
ein Momentenstützgriff (5) vorgesehen ist, der auf lösbare Weise drehfest mit dem Halsteil (2) verbindbar ist.

15. Modularer Femurkopfprothesensatz nach Anspruch 14,
**dadurch gekennzeichnet, dass**
der Momentenstützgriff (5) mit einer Aufnahme für einen Haltekonus (22) des Halsteils (2) versehen ist.

16. Modularer Femurkopfprothesensatz nach Anspruch 15,
**dadurch gekennzeichnet, dass**
an der Aufnahme eine Klemmeinrichtung (55) mit einem handbetätigten Exzenter (57) angeordnet ist.

17. Modularer Femurkopfprothesensatz nach einem der Ansprüche 13 bis 16,
**dadurch gekennzeichnet, dass**
ein drehmomentkalibrierter Anzugschlüssel (6) für das Verschraubungselement (3) vorgesehen ist.

## Claims

1. Modular femur-head prosthesis comprising a shaft part (1) for inserting into a medullary opening of a femur, and a neck part (2) with a receiving surface (25), which neck part (2) can be secured on a supporting surface (15) in the end region of the shaft part (1) by means of a screw element (3),
wherein
at least one separate intermediate ring (4) is provided, which is arranged between the supporting surface (15) of the shaft part (1) and the receiving surface (25) of the neck part (2) and is fixed by means of the screw element, and wherein the receiving surface (25) is arranged recessed in a depression that forms an enclosure around the intermediate ring (4), **characterized in that** the screw element is an expansion screw (3).

2. Modular femur-head prosthesis according to Claim 1,
**characterized in that**
the supporting and receiving surfaces (15, 25) are substantially plane.

3. Modular femur-head prosthesis according to one of the preceding claims,
**characterized in that**
the receiving surface (25) is formed at the bottom of a sleeve (24) whose inner jacket surrounds the proximal end of the shaft part (1).

4. Modular femur-head prosthesis according to Claim 3,
**characterized in that**
the outer jacket (14) of the proximal end of the shaft part (1) is designed as a guide surface.

5. Modular femur-head prosthesis according to one of the preceding claims,
**characterized in that**
the screw element has a thread area (32) which is longer than the thickness of the intermediate ring (4) .

6. Modular femur-head prosthesis according to one of the preceding claims,
**characterized in that**
several intermediate rings (4, 4', 4") with different thicknesses are provided.

7. Modular femur-head prosthesis according to Claim 6,
**characterized in that**
two intermediate rings with a division of the thicknesses in the ratio of 1:3 are provided.

8. Modular femur-head prosthesis according to Claim 6,
**characterized in that**
three intermediate rings (4, 4', 4") with a division of the thicknesses in the ratio of 5:2:1 are provided.

9. Modular femur-head prosthesis according to one of Claims 6 to 8,
**characterized in that**
several screw elements of different lengths are provided.

10. Modular femur-head prosthesis according to Claim 9,
**characterized in that**
the number of the different screw elements is smaller than the number of the combinations of the intermediate rings (4, 4', 4").

11. Modular femur-head prosthesis according to one of the preceding claims,
**characterized in that**
teeth (16) meshing in one another are provided on the supporting and receiving surfaces (15, 25).

12. Modular femur-head prosthesis according to Claim 11,
**characterized in that**
the teeth are radial teeth (16).

13. Modular femur-head prosthesis kit for forming a modular femur-head prosthesis according to one of Claims 1 to 12,
**characterized in that**
several intermediate rings (4, 4', 4") are provided in different thicknesses.

14. Modular femur-head prosthesis kit according to Claim 13,
**characterized in that**
a torque support grip (5) is provided which can be connected releasably and in a rotationally fixed manner to the neck part (2).

15. Modular femur-head prosthesis kit according to Claim 14,
**characterized in that**
the torque support grip (5) is provided with a seat for a retention cone (22) of the neck part (2) .

16. Modular femur-head prosthesis kit according to Claim 15,
**characterized in that**
a clamping device (55) with a manually actuated eccentric (57) is arranged on the seat.

17. Modular femur-head prosthesis kit according to one of Claims 13 to 16,
**characterized in that**
a torque-calibrated tightening wrench (6) is provided for the screw element (3).

## Revendications

1. Prothèse de tête fémorale modulaire comprenant une partie de tige (1) à introduire dans une ouverture médullaire d'un fémur et une partie de col (2) avec une face de réception (25), qui peut être fixée sur une face d'appui (15) dans la région d'extrémité de la partie de tige (1) au moyen d'un élément à visser (3), dans laquelle il est prévu au moins une bague intermédiaire séparée (4), qui est disposée entre la face d'appui (15) de la partie de tige (1) et la face de réception (25) de la partie de col (2) et qui est fixée au moyen de l'élément à visser, et dans laquelle la face de réception (25) est disposée en position enfoncée dans un creux contenant la bague intermédiaire (4), **caractérisée en ce que** l'élément à visser est une vis d'expansion (3).

2. Prothèse de tête fémorale modulaire selon la revendication 1, **caractérisée en ce que** les faces d'appui et de réception (15, 25) sont essentiellement planes.

3. Prothèse de tête fémorale modulaire selon l'une quelconque des revendications précédentes, **caractérisée en ce que** la face de réception (25) est formée sur le fond d'une douille (24), dont la surface latérale intérieure entoure l'extrémité proximale de la partie de tige (1).

4. Prothèse de tête fémorale modulaire selon la revendication 3, **caractérisée en ce que** la surface latérale extérieure (14) de l'extrémité proximale de la partie de tige (1) forme une face de guidage.

5. Prothèse de tête fémorale modulaire selon l'une quelconque des revendications précédentes, **caractérisée en ce que** l'élément à visser présente une zone filetée (32), qui est plus longue que l'épaisseur de la bague intermédiaire (4).

6. Prothèse de tête fémorale modulaire selon l'une quelconque des revendications précédentes, **caractérisée en ce qu'**il est prévu plusieurs bagues intermédiaires (4, 4', 4") avec des épaisseurs différentes.

7. Prothèse de tête fémorale modulaire selon la revendication 6, **caractérisée en ce qu'**il est prévu deux bagues intermédiaires avec une gradation des épaisseurs dans un rapport 1:3.

8. Prothèse de tête fémorale modulaire selon la revendication 6, **caractérisée en ce qu'**il est prévu trois bagues intermédiaires (4, 4', 4") avec une gradation des épaisseurs dans un rapport 5:2:1.

9. Prothèse de tête fémorale modulaire selon l'une quelconque des revendications 6 à 8, **caractérisée en ce qu'**il est prévu plusieurs éléments à visser de longueurs différentes.

10. Prothèse de tête fémorale modulaire selon la revendication 9, **caractérisée en ce que** le nombre des éléments à visser différents est inférieur au nombre des combinaisons des bagues intermédiaires (4, 4', 4").

11. Prothèse de tête fémorale modulaire selon l'une quelconque des revendications précédentes, **caractérisée en ce qu'**il est prévu sur les faces d'appui et de réception (15, 25) des dentures (16) engrenant l'une dans l'autre.

12. Prothèse de tête fémorale modulaire selon la revendication 11, **caractérisée en ce que** la denture est une denture radiale (16).

13. Ensemble de prothèse de tête fémorale modulaire pour la formation d'une prothèse de tête fémorale modulaire selon l'une quelconque des revendications 1 à 12, **caractérisé en ce qu'**il est prévu plusieurs bagues intermédiaires (4, 4', 4") avec des épaisseurs différentes.

14. Ensemble de prothèse de tête fémorale modulaire selon la revendication 13, **caractérisé en ce qu'**il est prévu une poignée de support de couple (5), qui peut être assemblée en rotation de façon séparable à la partie de col (2).

15. Ensemble de prothèse de tête fémorale modulaire selon la revendication 14, **caractérisé en ce que** la poignée de support de couple (5) est munie d'un logement pour un cône de maintien (22) de la partie de col (2).

16. Ensemble de prothèse de tête fémorale modulaire selon la revendication 15, **caractérisé en ce qu'**un dispositif de serrage (55) avec un excentrique (57) à actionnement manuel est disposé sur le logement.

17. Ensemble de prothèse de tête fémorale modulaire selon l'une quelconque des revendications 13 à 16, **caractérisé en ce qu'**il est prévu une clé de serrage à couple calibré (6) pour l'élément à visser (3).
